# EUROPÄISCHE PATENTSCHRIFT

(11) **EP 1 453 478 B1**
(45) Veröffentlichungstag und Bekanntmachung des Hinweises auf die Patenterteilung: **12.10.2011**
(21) Anmeldenummer: 02807682.6
(22) Anmeldetag: 01.08.2002
(51) Int. Cl.: A61K 8/37, A61K 8/29, A61K 8/34, A61K 8/35, A61K 8/40, A61K 8/58

(54) **KOSMETISCHE UND DERMATOLOGISCHE LICHTSCHUTZFORMULIERUNGEN MIT EINEM GEHALT AN PARTIKULÄREN UV-FILTERSUBSTANZEN UND ALKYLNAPHTHALATEN**
COSMETIC AND DERMATOLOGICAL LIGHT PROTECTIVE FORMULATIONS WITH A CONTENT OF PARTICULAR UV FILTER SUBSTANCES AND ALKYLNAPHTHALATES
FORMULATIONS COSMETIQUES ET DERMATOLOGIQUES DE PROTECTION SOLAIRE PRESENTANT UNE CERTAINE TENEUR EN SUBSTANCES PARTICULAIRES FILTRANT LES UV ET EN ALKYLNAPHTHALATES

(30) Priorität: 29.08.2001 DE 10141473
(43) Veröffentlichungstag der Anmeldung: 08.09.2004
(73) Patentinhaber: Beiersdorf AG, 20245 Hamburg (DE)
(72) Erfinder: WENDEL, Volker, 20255 Hamburg (DE); GÖPPEL, Anja, 22527 Hamburg (DE); SCHULZ, Jens, 22689 Schenefeld (DE)
(74) Vertreter: Rabanus, Birgit
(86) Internationale Anmeldenummer: PCT/EP2002/008577
(87) Internationale Veröffentlichungsnummer: WO 2004/056333

(56) Entgegenhaltungen:
- EP-A- 1 127 568
- EP-A- 1 127 569
- EP-A- 1 129 696
- WO-A-00/57850
- WO-A2-01/05361
- FR-A- 2 801 210
- FR-A- 2 801 213
- BONDA C ET AL: "A NEW PHOTOSTABILIZER FOR FULL SPECTRUM SUNCREENS" Juni 2000 (2000-06) , COSMETICS & TOILETRIES, WHEATON, IL, US, VOL. 115, NR. 6, PAGE(S) 37-45 XP001010090 ISSN: 0361-4387 Seite 38, rechte Spalte -Seite 39, linke Spalte Seite 41, linke Spalte -Seite 45
- EUREKALERT, [Online] 8. Juni 2001 (2001-06-08), XP002223321 Gefunden im Internet: <URL:http://sunsite1.dc.stanford.org/relea ses/acs-irr3060801.html> [gefunden am 2002-12-02]
- "Beauty is Skin Deep" HAPPI, [Online] September 2000 (2000-09), XP002223322 Gefunden im Internet: <URL:http://www.happi.com/special/sep002.h tlm> [gefunden am 2002-12-02]
- "Ethylendiamintetraessigsäure", RÖMPP Online, Version 3.6, 1 November 2003 (2003-11-01), Retrieved from the Internet: URL:http://www.roempp.com/prod/roempp.php [retrieved on 2010-05-17]
- "Mexoryl XL Sunblock", , Retrieved from the Internet: URL:http://antheliosonline.com/mexoryl-xl- article/ [retrieved on 2010-05-20]
- "Vergleichsversuch", ,

## Beschreibung

Die vorliegende Erfindung betrifft kosmetische und dermatologische Lichtschutzzubereitungen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich beispielsweise um Derivate des 3-Benzylidencamphers, der 4-Aminobenzoesäure, der Zimtsäure, der Salicylsäure, des Benzophenons sowie des Triazins handelt.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u. a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Homschicht zurückgehalten werden.

Es ist daher von grundsätzlicher Wichtigkeit, daß kosmetische und dermatologische Lichtschutzzubereitungen sowohl gegen UV-B- als auch gegen UV-A-Strahlung ausreichenden Schutz bieten.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen.

Auch mit Hilfe von organischen oder anorganischen partikulären UV-Filtersubstanzen ist ein effektiver UV-Schutz zu erreichen. Dabei nimmt der Schutzeffekt gegenüber UV-Strahlen zu, je kleiner die eingesetzten Partikel sind. Die Teilchenverkleinerung bewirkt gleichzeitig, daß sichtbares Licht durchgelassen wird, weshalb die Formulierungen transparent erscheinen; ein unerwünschter Weißeleffekt, durch den die betroffenen Hautpartien deutlich weiß gefärbt werden, tritt nur noch bei sehr hohen Partikelkonzentrationen beim anfänglichen Auftragen der Creme auf. Um zu einem wirksamen Sonnenschutz mit Hilfe von partikulären UV-Filtersubstanzen zu gelangen, ist es entscheidend, daß die Primärpartikel erhalten bleiben und die Dispersion stabil bleibt. Allerdings wird durch die Teilchenverkleinerung die Oberfläche der Partikel stark vergrößert, so daß diese aufgrund der zunehmenden Anziehungskräfte zur Zusammenballung neigen.

Eine Zusammenballung der Partikel führt aber zur Abnahme der galenischen Stabilität und zur Verringerung der UV-Schutzleistung der Formulierung sowie zur Zunahme des Streuvermögens im sichtbaren Bereich, damit also zum Weißeleffekt. Die Verarbeitung von Mikropigmenten erfordert deshalb eine optimale Formulierung und einen angepaßten Herstellungsprozeß.

Es war daher Aufgabe der vorliegenden Erfindung, die Nachteile des Standes der Technik zu beseitigen und auf einfache Weise zu stabilen Zubereitungen zu gelangen, welche sich durch eine hohe UV-Schutzleistung auszeichnen und in welchen eine feine Verteilung von partikulären UV-Filtersubstanzen vorliegt und erhalten bleibt.

Es war überraschend und für den Fachmann nicht vorauszusehen, daß lichtschutzwirksame kosmetische oder dermatologische Zubereitungen gemäß Anspruch 1 den Nachteilen des Standes der Technik abhelfen.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen (FR2801210). Solche Formulierungen können vorzugsweise auch eine Mikroemulsion, eine Feststoff-Emulsionen (d. h. eine Emulsion, welche durch Feststoffe stabilisiert ist, z. B. eine PickeringEmulsion), eine sprühbare Emulsion oder eine Hydrodispersion sein.

Die erfindungsgemäßen Zubereitungen stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische und kosmetische Eigenschaften, wie beispielsweise die Verteilbarkeit auf der Haut oder das Einzugsvermögen in die Haut, und zeichen sich ferner durch eine sehr gute Lichtschutzeffektivität bei gleichzeitig hervorragenden Hautpflegedaten aus.

Die UV-Schutzleistung von Sonnenschutzmitteln bzw. der ihnen zugrunde liegenden UV-Filter wird in der Regel in biologischen Wirksamkeitsprüfungen unter standardisierten Bedingungen bestimmt. Mit "UV-Schutzleistung" ist im Sinne der vorliegenden Erfindung sowohl die Schutzleistung gegenüber UV-A-Strahlung als auch gegenüber UV-B-Strahlung gemeint.

Ein Maß für die UV-Schutzleistung stellen im Sinne der vorliegenden Erfindung beispielsweise der Lichtschutzfaktor (LSF bzw. SPF) oder auch IPD-Werte und dergleichen dar.

Der Lichtschutzfaktor (LSF, oft auch SPF (sun protection factor) genannt) gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich von 320 bis 360 nm absorbieren.

Bevorzugte partikuläre UV-Filtersubstanzen im Sinne der vorliegenden Erfindung sind anorganische Pigmente, insbesondere Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| ZnO Neutral | / | H&R |
| MZ- 300 | / | Tayca Corporation |
| MZ- 500 | / | Tayca Corporation |
| MZ- 700 | / | Tayca Corporation |
| MZ- 303S | 3% Methicone | Tayca Corporation |
| MZ- 505S | 5% Methicone | Tayca Corporation |
| MZ- 707S | 7% Methicone | Tayca Corporation |
| MZ- 303M | 3% Dimethicone | Tayca Corporation |
| MZ- 505M | 5% Dimethicone | Tayca Corporation |
| MZ- 707M | 7% Dimethicone | Tayca Corporation |
| Z- Sperse Ultra | ZnO (>=56%) / Ethylhexyl | Collaborative |
| | Hydroxystearate Benzoate / Di- | Laboratories |
| | methicone/ Cyclomethicone | |
| Samt- UFZO- | ZnO (60%) / Cyclomethicone / | Miyoshi Kasei |
| 450/D5 (60%) | Dimethicone | |

Besonderes bevorzugte Zinkoxide im Sinne der Erfindung sind das Z-Cote HP1 und das Z-Cote von der Firma BASF und das Zinkoxid NDM von der Firma Haarmann & Reimer.

Erfindungsgemäße Titandioxid-Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen. Erfindungsgemäße beschichtete und unbeschichtete Titandioxide können in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Geeignete Titandioxidpartikel und Vordispersionen von Titandioxidpartikein im Sinne der vorliegenden Erfindung sind unter folgenden Handelsbezeichnungen bei den aufgeführten Firmen erhältlich:

| **Handelsname** | **Coating** | **zusätzliche Bestandteile der Vordispersion** | **Hersteller** |
|---|---|---|---|
| MT-150W | None | - | Tayca Corporation |
| MT-150A | None | - | Tayca Corporation |
| MT-500B | None | - | Tayca Corporation |
| MT-600B | None | - | Tayca Corporation |
| MT-100TV | Aluminiumhydroxid | - | Tayca Corporation |
| | Stearinsäure | | |
| MT-100Z | Aluminiumhydroxid | - | Tayca Corporation |
| | Stearinsäure | | |
| MT-100T | Aluminiumhydroxid | - | Tayca Corporation |
| | Stearinsäure | | |
| MT-500T | Aluminiumhydroxid | - | Tayca Corporation |
| | Stearinsäure | | |
| MT-100S | Aluminiumhydroxid | - | Tayca Corporation |
| | Laurinsäure | | |
| MT-100F | Stearinsäure | - | Tayca Corporation |
| | Eisenoxid | | |
| MT-100SA | Alumina | - | Tayca Corporation |
| | Silica | | |
| MT-500SA | Alumina | - | Tayca Corporation |
| | Silica | | |
| MT-600SA | Alumina | - | Tayca Corporation |
| | Silica | | |
| MT-100SAS | Alumina | - | Tayca Corporation |
| | Silica | | |
| | Silikon | | |
| MT-500SAS | Alumina | - | Tayca Corporation |
| | Silica | | |
| | Silikon | | |
| MT-500H | Alumina | - | Tayca Corporation |
| MT-100AQ | Silica | - | Tayca Corporation |
| | Aluminiumhydroxid | | |
| | Alginsäure | | |
| Eusolex T | Wasser | - | Merck KgaA |
| | Simethicone | | |
| Eusolex T-2000 | Alumina | | Merck KgaA |
| | Simethicone | | |
| Eusolex T-Olio F | Silica Dimethylsilate | C₁₂₋₁₅ Alkylbenzoate | Merck KgaA |
| | Wasser | Calcium Poly- | |
| | | hydroxystearate | |
| | | Silica Dimethylsilate | |
| Eusolex T-Olio P | Wasser | Octyl Palmitate | Merck KgaA |
| | Simethicone | PEG-7 | |
| | | Hydrogenated Castor | |
| | | Oil | |
| | | Sorbitan Oleate | |
| | | Hydrogenated Castor | |
| | | Oil | |
| | | Beeswax | |
| | | Stearinsäure | |
| Eusolex T-Aqua | Wasser | Phenoxyethanol | Merck KgaA |
| | Alumina | Natrium | |
| | Natriummetaphosphat | Methylparabene | |
| | | Natriummetaphosph | |
| | | at | |
| Eusolex T-45D | Alumina | Isononyl | Merck KgaA |
| | Simethicone | Isononanuate | |
| | | Polyglyceryl | |
| | | Ricinoleate | |
| Kronos 1171 | None | - | Kronos |
| (Titandioxid 171) | | | |
| Titandioxid P25 | None | - | Degussa |
| Titandioxid T805 | Octyltrimethylsilan | - | Degussa |
| (Uvinul TiO₂) | | | |
| UV-Titan X610 | Alumina | - | Kemira |
| | Dimethicone | | |
| UV-Titan X170 | Alumina | - | Kemira |
| | Dimethicone | | |
| UV-Titan X161 | Alumina | - | Kemira |
| | Silica | | |
| | Stearinsäure | | |
| UV-Titan M210 | Alumina | - | Kemira |
| UV-Titan M212 | Alumina | Glycerol | Kemira |
| UV-Titan M262 | Alumina | - | Kemira |
| | Silikon | | |
| UV-Titan M160 | Alumina | - | Kemira |
| | Silica | | |
| | Stearinsäure | | |
| Tioveil AQ 10PG | Alumina | Wasser | Solaveil |
| | Silica | Propylenglycol | Uniquema |
| Mirasun TiW 60 | Alumina | Wasser | Rhone-Poulenc |
| | Silica | | |

Die erfindungsgemäßen Titandioxide zeichnen sich durch eine Primärpartikelgröße zwischen 10 nm bis 150 nm aus.

Im Sinne der vorliegenden Erfindung sind besonders bevorzugte Titandioxide das MT-100 Z und MT-100 TV von Tayca Corporation, Eusolex T-2000 von Merck und das Titandioxid T 805 von Degussa.

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCl:Bisoctyltriazol], welches unter der Handelsbezeichnung Tinosorb® M bei der CIBA-Chemikalien GmbH erhältlich ist.

Es ist insbesondere vorteilhaft im Sinne der vorliegenden Erfindung, partikülare UV-Filtersubstanzen, welche nicht bereits in Form einer Vordispersion vorliegen, zunächst in einem oder mehreren erfindungsgemäßen Dialkylnaphthalaten zu dispergieren und diese Grunddispersion anschließend weiterzuverarbeiten. Während kommerziell erhältliche Vordispersionen in der Regel mit Hilfsstoffen zur Stabilisierung versetzt sind, welche mit weiteren Substanzen der kosmetischen oder dermatologischen Formulierung unerwünschte Wechselwirkungen eingehen können, kann bei der Herstellung erfindungsgemäßer Grunddispersionen erstaunlicherweise auf die Zugabe derartiger Stabilisatoren verzichtet werden.

Die Gesamtmenge an einer oder mehreren partikulären UV-Filtersubstanzen in den fertigen kosmetischen oder dermatologischen Zubereitungen wird vorteilhaft aus dem Bereich 0,01 Gew.-% bis 20 Gew.-%, vorzugsweise von 0,1 bis 10 Gew.-% gewählt, jeweils bezogen auf das Gesamtgewicht der Zubereitungen.

Vorteilhaft im Sinne der vorliegenden Erfindung sind Dialkylnaphthalate, für die R¹ und/oder R² verzweigte Alkylgruppen mit 6 bis 10 Kohlenstoffatomen darstellen. Ganz besonders bevorzugt im Sinne der vorliegenden Erfindung ist Diethylhexylnaphthalat, welches beispielsweise unter der Handelsbezeichnung Hallbrite TQ™ von CP Hall oder Corapan TQ™ von H&R erhältlich ist.

Erfindungsgemäß enthalten kosmetische oder dermatologische Zubereitungen 0,001 bis 20 Gew.-%, vorteilhaft 0,01 bis 15 Gew.-%. ganz besonders bevorzugt 3 bis 10 Gew.-% eines oder mehrerer Dialkylnaphthalate.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern. Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Erfindungsgemäße Zubereitungen werden erhalten, wenn als Zusatz- oder Wirkstoffe Antioxidantien eingesetzt werden. Erfindungsgemäß enthalten die Zubereitungen eines oder mehrere Antioxidantien. Als günstige Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Camitin, Camosin, natürliche und/oder synthetische Isoflavonoide, Kreatin, Taurin und/oder ß-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Auch Moisturizer können bevorzugt verwendet werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Homschicht (auch transepidermal water loss (TEWL) genannt) zu reduzieren und/oder die Hydratation der Homschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registratumummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bomitrid etc.) und/oder Aerosile^{®} (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkemöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*).

Auch beliebige Abmischungen solcher Öl-und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 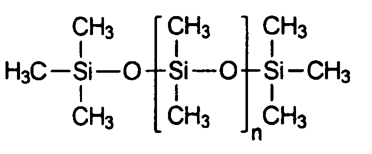 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescrèmes oder Makeup-Produkten gewöhnlich UV-A- bzw. UV-B-Filtersubstanzen eingearbeitet Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B-und/oder Breitbandfiltersubstanz.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz und/oder ein oder mehrere Silikonderivate als weitere Phase enthalten. Ölfreie Formulierungen im Sinne der vorliegenden Erfindung können vorteilhaft auch weitere lipophile Komponenten - wie beispielsweise lipophile Wirkstoffe - enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Vorteilhafte weitere UV-Filtersubstanzen im Sinne der vorliegenden Erfindung sind sulfonierte, wasserlösliche UV-Filter, wie z. B.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bisnatriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bomylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenrnethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Methylene Bis-Benzotriazolmethylbutylphenol), welches unter der Handelsbezeichnung Tinosorb® S bei der CIBA-Chemikalien GmbH erhältlich ist;
- Dioctylbutylamidotriazon (INCI: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich sein. Vorteilhafte öllösliche Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Eine weiterere erfingdungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-([4-(2-Ethyl-hexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVPNA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP)

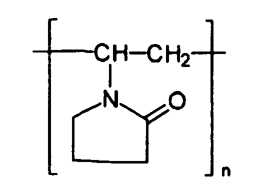

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### 1. O/W Sonnenschutz Emulsionen

| | **1** | **2** | **3** | **4** |
|---|---|---|---|---|
| Glycerinmonostearat SE | 0,50 | 3,00 | | |
| Glyceryl Stearat Citrat | 2,00 | | 2,00 | 2,50 |
| Stearinsäure | | | | |
| PEG-40 Stearat | 0,50 | | | |
| Cetyl Phosphat | | | 1,00 | |
| Stearyl Alkohol | | 3,00 | | 0,50 |
| Cetyl Alkohol | 2,50 | | 0,50 | 2,00 |
| Ethylhexyl Methoxycinnamat | | | 6,00 | 8,00 |
| Bis-Ethylhexyloxyphenol | | | 2,50 | 2,50 |
| methoxyphenyl Triazin | | | | |
| Butyl Methoxydibenzoylmethan | 1,00 | 2,00 | 2,80 | 1.50 |
| Dinatrium Phenyl | 2,50 | 0,50 | 1,00 | 0,30 |
| Dibenzimidazol Tetrasulfonat | | | | |
| Ethylhexyl Triazon | 4,00 | 3,00 | 4,00 | |
| 4-Methylbenzyliden Campher | 4,00 | | 2,00 | 2,00 |
| Octocrylen | | | | 2,50 |
| Diethylhexyl Butamido Triazon | 1,00 | | 1,00 | |
| Phenylbenzmidazol | 0,50 | | | |
| Sulfonsäure | | | | |
| Methylen Bis-Benzotriazolyl | 2,00 | 0,50 | 2,50 | |
| Tetramethylbutylphenol | | | | |
| Benzophenon-3 | | | | |
| Isoamyl p-Methoxyzinnamat | | | | |
| Homosalat | | | | |
| Ethylhexylsalicylat | | 3,00 | | 5,00 |
| Drometrizol Trisiloxan | | 0,5 | | |
| Terephthaliden Dicamphor | | | 1,00 | |
| Sulfonsäure | | | | |
| Diethylhexyl-2,6-naphthalat | 3,50 | 7,00 | 6,70 | 8,00 |
| Titandioxid MT-100Z | | | | |

| | | | | | | | |
|---|---|---|---|---|---|---|---|
| Titandioxid MT-100TV | 5,0 | | 10,0 | | 2,00 | | |
| Titandioxid Eusolex T-2000 | 2,0 | | 2,0 | 7,0 | | | 10,0 |
| Titandioxid T805 | | 3,0 | | | 2,00 | 2,00 | |
| Titandioxid Eusolex T-Aqua | | | 2,0 | | | | 5,5 |
| Zinkoxid HP1 | | | 1,50 | | | | 4,0 |
| Zinkoxid NDM | 1,0 | | | | | 8,0 | |
| Zinkoxid Neutral | | 5,0 | | 8,0 | 10,0 | | 4,0 |
| Zinkoxid MZ-303S | 5,0 | | 2,5 | | | | |
| C12-15 Alkyl Benzoat | | 2,50 | | | 4,00 | 7,00 | 5,00 |
| Dicaprylyl Ether | | | 3,50 | | 2,00 | | |
| Butylenglycol | 5,00 | | | 6,00 | | | |
| Dicaprylat/Dicaprat | | | | | | | |
| Dicaprylyl Carbonat | | | 6,00 | | | 2,00 | 2,00 |
| Dimethicon | | 0,50 | 1,00 | | 2,00 | | |
| Cyclomethicon | 2,00 | | | 0,50 | | | 0,50 |
| Shea Butter | | 2,00 | | | | | 0,50 |
| PVP Hexadecen Copolymer | 0,50 | | | 0,50 | 1,00 | | 1,00 |
| Tricontanyl PVP | | 0,50 | 1,00 | | | | 1,00 |
| Glycerin | 3,00 | 7,50 | | 7,50 | 5,00 | | 2,50 |
| Xanthan Gummi | 0,15 | | 0,05 | | | | 0,30 |
| Sodium Carbomer | | 0,20 | 0,10 | 0,20 | | | |
| Vitamin E Acetat | 0,50 | | 0,25 | | 0,75 | | 1,00 |
| Polyurethan | | 0,50 | | 0,50 | | 1,00 | |
| Styrene/Acrylat Copolymer | 0.80 | | 3.00 | 1.50 | | | |
| DMDM Hydantoin | | 0,60 | 0,40 | 0,20 | | | |
| Konkaben LMB ® | | | | 0,18 | 0,20 | 0,10 | 0,15 |
| Methylparaben | 0,15 | | 0,25 | | 0,50 | | |
| Phenoxyethanol | 1,00 | 0,40 | | 0,40 | 0,50 | 0,40 | 0,60 |
| Ethanol | | 2,00 | 1,50 | | 3,00 | | 1,00 |
| Parfüm | 0,20 | 0,20 | 0,20 | 0,20 | 0,20 | 0,20 | 0,20 |
| Wasser | ad | ad | ad | ad | ad | ad | ad |
| | 100 | 100 | 100 | 100 | 100 | 100 | 100 |

### 2. Hydrodispersionen

| | 1 | 2 | 3 |
|---|---|---|---|
| Ceteareth-20 | 1,00 | | |
| Cetyl Alkohol | | 1,00 | |
| Sodium Carbomer | | | |
| Acrylates/C10-30 Alkyl Acrylat | 0,50 | 0,40 | 0,10 |
| Crosspolymer | | | |
| Xanthan Gummi | | 0,15 | 0,50 |
| Ethylhexyl Methoxycinnamat | | | 8,00 |
| Bis-Ethylhexyloxyphenol | | 2,00 | 2,50 |
| methoxyphenyl Triazin | | | |
| Butyl Methoxydibenzoylmethan | 1,00 | 2,00 | 2,50 |
| Dinatrium Phenyl Dibenzimidazol | 0,50 | 1,50 | 3.00 |
| Tetrasulfonat | | | |
| Ethylhexyl Triazon | 4,00 | 3,00 | |
| 4-Methylbenzyliden Camphor | 4,00 | | 2,00 |
| Octocrylen | | 3,90 | 2,50 |
| Diethyhexyl Butamido Triazon | 1,00 | | |
| Phenylbenzmidazol Sulfonsäure | 0,50 | | |
| Methylen Bis-Benzotriazolyl | 2,50 | | 0,80 |
| Tetramethylbuthylphenol | | | |
| Drometrizol Trisiloxan | | 1,00 | 1,50 |
| Terephthaliden Dicamphor | | | 1,00 |
| Sulfonsäure | | | |
| Diethylhexyl-2,6-naphthalat | 4,50 | 7,20 | 9,80 |
| Titandioxid MT-100Z | 0,50 | 2,00 | 1,00 |
| Titandioxid MT-100TV | | | |
| Titandioxid Eusolex T-2000 | | | |
| Titandioxid T805 | 5,5 | | 7,5 |
| Titandioxid Eusolex T-Aqua | | 4,5 | |
| Zinkoxid HP1 | 2,0 | | |
| Zinkoxid NDM | | | 5,5 |
| Zinkoxid Neutral | 1,0 | | 0,5 |
| Zinkoxid MZ-303S | | | |
| C12-15 Alkyl Benzoat | 2,00 | | |
| Dicaprylyl Ether | | | |
| Butylenglycol Dicaprylat/Dicaprat | 4,00 | 2,00 | |
| Dicaprylyl Carbonat | | 6,00 | |
| Dimethicon | | 1,00 | |
| Phenyltrimethicon | 2,00 | | 2,00 |
| Shea Butter | | | |
| PVP Hexadecen Copolymer | 0,50 | | 1,00 |
| Tricontanyl PVP | 0,50 | 1,00 | |
| Ethylhexylglycerin | | 1,00 | 0,50 |
| Glycerin | 3,00 | 7,50 | 2,50 |
| Glycin Soja | | 1,50 | |
| Vitamin E Acetat | 0,50 | 0,25 | 1,00 |
| Polyurethan | | 1,50 | |
| Styrene/Acrylat Copolymer | | 0.50 | 2.00 |
| DMDM Hydantoin | | 0,40 | |
| Konkaben LMB ® | 0,20 | | 0,15 |
| Methylparaben | 0,50 | 0,25 | |
| Phenoxyethanol | 0,50 | | 0,60 |
| Ethanol | 3,00 | 1,50 | 1,00 |
| Parfüm | 0,20 | 0,20 | 0,20 |
| Wasser | ad 100 | ad 100 | ad 100 |

### 3. W/O Sonnenschutz Emulsionen

| | 1 | 2 | 3 |
|---|---|---|---|
| CetyldimethiconCopotyol Cetyldimethicon Copolyol | | | |
| Polyglyceryl-2- | 5,00 | | 4,50 |
| dipolyhydroxystearat | | | |
| PEG-30-dipolyhydroxystearat | | 5,00 | |
| Ethylhexyl Methoxycinnamat | | | 4,00 |
| Bis-Ethylhexyloxyphenol | 2,00 | | 2,50 |
| Methoxyphenyl Triazin | | | |
| Butyl Methoxydibenzoylmethan | 0.50 | 2,00 | 0,70 |
| Dinatrium Phenyl Dibenzimidazol | 0,50 | 1.60 | 2.60 |
| Tetrasulfonat | | | |
| Ethylhexyl Triazon | | 3,00 | |
| 4-Methylbenzyliden Camphor | | | 2,00 |
| Octocrylen | 0,90 | 3,90 | 2,50 |
| Diethyhexyl Butamido Triazon | 1,00 | | |
| Phenylbenzmidazol Sulfonsäure | 0,50 | | 2,00 |
| Methylen Bis-Benzotriazolyl | | 2,00 | |
| Tetramethylbutylphenol | | | |
| Drometrizol Trisiloxan | | | 1,50 |
| Terephthaliden Dicamphor | | 1,00 | 0,50 |
| Sulfonsäure | | | |
| Diethylhexyl-2,6-naphthalat | 7.50 | 3.50 | 9.70 |
| Titandioxid MT-100Z | 3,0 | | 1,5 |
| Titandioxid MT-100TV | | | 2,5 |
| Titandioxid Eusolex T-2000 | | 7,5 | |
| Titandioxid T805 | 2,0 | 8,0 | |
| Titandioxid Eusolex T-Aqua | | 0,5 | 10,0 |
| Zinkoxid HP1 | 2,0 | | |
| Zinkoxid NDM | | | |
| Zinkoxid Neutral | | 1,50 | |
| Zinkoxid MZ-303S | 1,00 | 2,5 | 2,00 |
| Mineralöl | | 10,0 | 8,00 |
| C12-15 Alkyl Benzoat | | | |
| Dicaprylyl Ether | 10,00 | | 7,00 |
| Butylenglycol Dicaprylat/Dicaprat | | 2,00 | 4,00 |
| Dicaprylyl Carbonat | 5,00 | 6,00 | |
| Dimethicon | | 1,00 | |
| Cyclomethicon | 2,00 | | 2,00 |
| Shea Butter | | 3,00 | |
| PVP Hexadecen Copolymer | 0,50 | | 1,00 |
| Tricontanyl PVP | | 0,50 | 0,50 |
| Ethylhexylglycerin | | 1,00 | 0,50 |
| Glycerin | 3,00 | | 2,50 |
| Glycine Soja | | 1,50 | |
| MgSO₄ | 1,00 | | |
| MgCl₂ | | 1,00 | 0,70 |
| Vitamin E Acetat | 0,50 | 0,25 | 1,00 |
| Styrene/Acrylat Copolymer | 0.50 | | |
| Polyurethan | | | |
| DMDM Hydantoin | | 0,40 | |
| Methylparaben | 0,50 | 0,25 | |
| Phenoxyethanol | 0,50 | | 0,60 |
| Ethanol | 3,00 | 1,50 | 1,00 |
| Parfüm | 0,20 | 0,20 | 0,20 |
| Wasser | ad 100 | ad 100 | ad 100 |

**4. Feststoffstabilisierte Emulsionen**

## Patentansprüche

1. Lichtschutzwirksame kosmetische oder dermatologische Zubereitungen, **dadurch gekennzeichnet, daß** sie
(a) mindestens eine partikuläre UV-Filtersubstanz,
(b) mindestens ein Dialkylnaphthalat, welches sich durch die Strukturformel 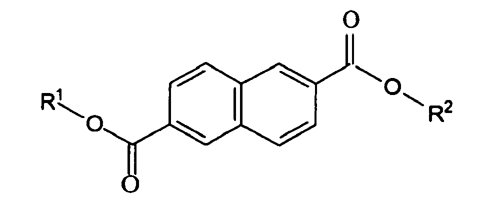 auszeichnet,
worin R¹ und R² unabhängig voneinander gewählt werden aus der Gruppe der verzweigten und unverzweigten Alkylgruppen mit 6 bis 24 Kohlenstoffatomen und
(c) eines oder mehrere Antioxidantien
enthalten, mit Ausnahme der folgenden Zubereitungen:
| | W/O-Pickering Emulsion |
|---|---|
| Phenyltrimethicon | 5,00 |
| Vitamin E Acetat | 0,50 |
| Drometrizole Trisiloxane | 5,00 |
| Bisoctyltriazol | 5,00 |
| Eusolex T2000® | 4,00 |
| Aerosil R972® | 1,00 |
| Halbrite TQ® | 4,00 |
| Konservierung | 0,50 |
| Glycerin | 3,00 |
| Wasser | Ad 100,00 |
| Phenyltrimethicon | 5,00 |
| Vitamin E Acetat | 0,50 |
| Dioctylbutamidotriazon | 5,00 |
| Aniso Triazin | 5,00 |
| Eusolex T2000® | 4,00 |
| Aerosil R972® | 1,00 |
| Halbrite TQ® | 4,00 |
| Konservierung | 0,50 |
| Glycerin | 3,00 |
| Wasser | Ad 100,00 |
| | O/W-Emulsion |
|---|---|
| Stearinsäure | 1,50 |
| Glycerinmonostearat | 3,00 |
| Dimethicon | 2,00 |
| Phenyltrimethicon | 2,00 |
| Vitamin E-Acetat | 0,50 |
| Dioctylbutamidotriazon | 3,00 |
| Octocrylen | 1,50 |
| Octylsalicylat | 5,00 |
| Butyl Methoxydibenzoyl Methan | 2,00 |
| Eusolex T2000®¹ | 1,00 |
| Hallbrite TQ®² | 4,00 |
| Konservierung | 0,50 |
| Glycerin | 3,00 |
| Xanthan Gum | 0,30 |
| Natronlauge 45% | 0,50 |
| Wasser | ad. 100,00 |
| | W/O Pickeringemulsion |
|---|---|
| Phenyltrimethicon | 5,00 |
| Vitamin E-Aceatat | 0,50 |
| Dioctylbutamidotriazon | 5,00 |
| Aniso Triazin | 5,00 |
| Octocrylen | 2,90 |
| Octylsalicylat | 5,00 |
| Octylmethoxycinnamat | 10,00 |
| Butylmethoxydibenzoylmethan | 3,00 |
| Eusolex T2000®¹ | 5,00 |
| Aerosil R972®² | 1,00 |
| Halbrite TQ®³ | 6,00 |
| Konservierung | 0,50 |
| Glycerin | 3,00 |
| Wasser | ad. 100 |
| | |
|---|---|
| ¹mit Simethicone und Aluminia beschichtete Titandioxidpigmente ² Diethylhexylnaphthalat ¹mit Simethicone und Aluminia beschichtete Titandioxidpigmente ² wasserabweisend beschichtete Siliciumdioxidpartikel ³Diethylhexylnaphthalat. | |

2. Zubereitung nach Anspruch 1, **dadurch gekennzeichnet, daß** der Gehalt an einem oder mehreren Dialkylnaphthalaten aus dem Bereich von 0,001 bis 20 Gew.-%, vorteilhaft 0,01 bis 15 Gew.%, besonders bevorzugt 3 bis 10 Gew.-% gewählt wird, bezogen auf das Gesamtgewicht der Zubereitung.

3. Zubereitung nach einem der vorhergehenden Ansprüche, **dadurch gekennzeichnet, daß** die partikuläre(n) UV-Filtersubstanz(en) aus der Gruppe der mikronisierten anorganische Pigmente, insbesondere der mikronisierten Metalloxide gewählt wird.

4. Zubereitung nach einem der vorhergehenden Ansprüche, **dadurch gekennzeichnet, daß** sie mindestens eine weitere UV-Filtersubstanz, gewählt aus der Gruppe Triazine, Benzotriazole und organische und/oder anorganische Pigmente, enthält.

5. Zubereitung nach einem der vorhergehenden Ansprüche, **dadurch gekennzeichnet, daß** sie mindestens eine weitere UV-A-Filtersubstanz und/oder einen Breitbandfilter, gewählt aus der Gruppe Dibenzoylmethanderivate [insbesondere das 4-(tert.-Butyl)-4'-methoxydibenzoylmethan] und 2,4-Bis-{[4-(2-Ethyl-hexyloxy)-2-hydroxy]-phenyl}-6-(4-methoxyphenyl)-1,3,5-triazin, enthält, wobei die weiteren Filtersubstanzen jeweils einzeln oder in beliebigen Kombinationen miteinander vorliegen können.

## Claims

1. Cosmetic or dermatological preparations which are effective for light protection, **characterized in that** they comprise
(a) at least one particulate UV filter substance,
(b) at least one dialkyl naphthalate having the structural formula 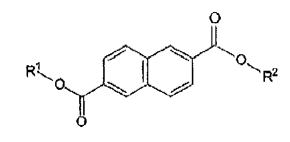 in which R¹ and R² are selected independently of one another from the group of branched and unbranched alkyl groups having 6 to 24 carbon atoms and
(c) one or more antioxidants,
excepting the following preparations.
| | W/O Pickering emulsion |
|---|---|
| Phenyltrimethicone | 5.00 |
| Vitamin E acetate | 0.50 |
| Drometrizole trisiloxane | 5.00 |
| Bisoctyltriazole | 5.00 |
| Eusolex T2000® | 4.00 |
| Aerosil R972® | 1.00 |
| Hallbrite TQ® | 4.00 |
| Preservation | 0.50 |
| Glycerol | 3.00 |
| Water | ad 100.00 |
| | W/O Pickering emulsion |
|---|---|
| Phenyltrimethicone | 5.00 |
| Vitamin E acetate | 0.50 |
| Dioctylbutamidotriazone | 5.00 |
| Aniso triazine | 5.00 |
| Eusolex T2000® | 4.00 |
| Aerosil R972® | 1.00 |
| Mallbrite TQ® | 4.00 |
| Preservation | 0.50 |
| Glycerol | 3.00 |
| Water | ad 100.00 |
| | O/W emulsion |
|---|---|
| Stearic acid | 1.50 |
| Glycerol monostearate | 3.00 |
| Simethicone | 2.00 |
| Phenyltrimethicone | 2.00 |
| Vitamin E acetate | 0.50 |
| Dioctylbutamidotriazone | 3.00 |
| Octocrylene | 1.50 |
| Octyl salicylate | 5.00 |
| Butyl methoxydibenzoyl methane | 2.00 |
| Eusolex T2000®¹ | 1.00 |
| Hallbrite TQ®² | 4.00 |
| Preservation | 0.50 |
| Glycerol | 3.00 |
| Xanthan Gum | 0.30 |
| Aqueous sodium hydroxide solution 45% | 0.50 |
| Water | ad 100.00 |
| | |
|---|---|
| ¹titanium dioxide pigments coated with simethicone and alumina ²diethylhexyl naphthalate | |
| | O/W Pickering emulsion |
|---|---|
| Fhenyltrimethicone | 5.00 |
| Vitamin E acetate | 0.50 |
| Dioctylbutamidotriazone | 5.00 |
| Aniso triazine | 5.00 |
| Octocrylene | 2.90 |
| Octyl salicylate | 5.00 |
| Octyl methoxycinnamate | 10.00 |
| Butyl methoxydibenzoyl methane | 3.00 |
| Eusolex T2000®¹ | 5.00 |
| Aerosil R972®² | 1.00 |
| Hallbrite TQ®³ | 6.00 |
| Preservation | 0.50 |
| Glycerol | 3.00 |
| Water | ad 100.00 |
| | |
|---|---|
| ¹titanium dioxide pigments coated with simethicone and alumina ²silicon dioxide particles coated to repel water ³diethylhexyl naphthalate | |

2. Preparation according to Claim 1, **characterized in that** the content of one or more dialkyl naphthalates is selected from the range from 0.001 to 20% by weight, advantageously 0.01 to 15% by weight, particularly preferably 3 to 10% by weight, based on the total weight of the preparation.

3. Preparation according to either of the preceding claims, **characterized in that** the particulate UV filter substance(s) is selected from the group of micronized inorganic pigments, in particular of micronized metal oxides.

4. Preparation according to any of the preceding claims, **characterized in that** it comprises at least one further UV filter substance chosen from the group of triazines, benzotriazoles and organic and/or inorganic pigments.

5. Preparation according to any of the preceding claims, **characterized in that** it comprises at least one further UV-A filter substance and/or a broad-band filter chosen from the group of dibenzoylmethane derivatives [in particular 4-(tert-butyl)-4'-methoxydibenzoylmethane] and 2,4-bis{[4-(2-ethylhexyloxy)-2-hydroxy]phenyl)-6-(4-methvxyphenyl)-1,3,5-triazine, where the further filter substances may be present each singly or in any combinations with one another.

## Revendications

1. Préparations cosmétiques ou dermatologiques, actives en protection contre la lumière, **caractérisées en ce qu'**elles contiennent
(a) au moins une substance filtre des UV sous forme de particules,
(b) au moins un naphtalate de dialkyle qui se distingue par la formule développée 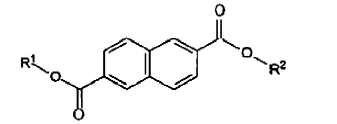 où R¹ et R² sont choisis indépendamment l'un de l'autre dans le groupe formé par les groupes alkyle ramifiés et non ramifiés comprenant 6 à 24 atomes de carbone, et
(c) un ou plusieurs antioxydants, à l'exception des préparations suivantes :
| | Mulsion de Pickering |
|---|---|
| | E/H |
| Phényltriméthicone | 5,00 |
| Acétate de vitamine E | 0,50 |
| Drométrizole Trisiloxane | 5,00 |
| Bisoctyltriazole | 5,00 |
| Eusolex T2000® | 4,00 |
| Aerosil R972® | 1,00 |
| Halbrite TQ® | 4,00 |
| Conservateur | 0,50 |
| Glycérol | 3,00 |
| Eau ad | 100,00 |
| | Emulsion de Pickering |
|---|---|
| | E/H |
| Phényltriméthicone | 5,00 |
| Acétate de vitamine E | 0,50 |
| Dioctylbutylamidotriazone | 5,00 |
| Anisotriazine | 5,00 |
| Eusolex T2000® | 4,00 |
| Aerosil R972® | 1,00 |
| Halbrite TQ® | 4,00 |
| Conservateur | 0,50 |
| Glycérol | 3.00 |
| Eau | ad 100,00 |
| | Emulsion H/E |
|---|---|
| Acide stéarique | 1,50 |
| Monostéarate de glycérol | 3,00 |
| Diméthicone | 2,00 |
| Phényltriméthicone | 2,00 |
| Acétate de vitamine E | 0,50 |
| Dioctylbutylamidotriazone | 3,00 |
| Octocrylène | 1,50 |
| Salicylate d'octyle | 5,00 |
| Eutylméthoxydibenzoylméthane | 2,00 |
| Eusolex T2000®¹ | 1,00 |
| Halbrite TQ®² | 4,00 |
| Conservateur | 0,50 |
| Glycérol | 3,00 |
| Gomme de xanthane | 0,30 |
| Lessive de soude caustique 45% | 0,50 |
| Eau | ad 100,00 |
| | |
|---|---|
| ¹ pigments de dioxyde de titane revêtus de Simethicone et d'alumine ² naphtalate de diéthylhexyle | |
| | Emulsion de Pickering |
|---|---|
| | E/H |
| Phényltriméthicone | 5,00 |
| Acétate de vitamine E | 0,50 |
| Dioctylbutylamidotriazone, | 5,00 |
| Anisotriazine | 5,00 |
| Octocrylène | 2,90 |
| Salicylate d'octyle | 5,00 |
| Méthoxycinnamate d'octyle | 10,00 |
| Butylméthoxydibenzoylméthane | 3,00 |
| Fusolex T2000®¹ 5,00 | |
| Aerosil R972®² | 1,00 |
| Halbrite TQ®³ | 6,00 |
| Conservateur | 0,50 |
| Glycérol 3,00 | |
| Eau ad | 100 |
| | |
|---|---|
| ¹ pigments de dioxyde de titane revêtus de Siméthicone et d'alumine ² particules de dioxyde de silicium revêtues de manière hydrofuge ³ naphtalate de diéthylhexyle. | |

2. Préparation selon la revendication 1, **caractérisée en ce que** la teneur en un ou plusieurs naphtalates de dialkyle est choisie dans la plage de 0,001 à 20% en poids, avantageusement de 0,01 à 15% en poids, de manière particulièrement préférée de 3 à 10% en poids, par rapport au poids total de la préparation.

3. Préparation selon l'une quelconque des revendications précédentes, **caractérisée en ce que** la/les substance(s) filtre des UV sous forme de particules est/sont choisie(s) dans le groupe formé par les pigments inorganiques micronisés, en particulier les oxydes de métal micronisés.

4. Préparation selon l'une quelconque des revendications précédentes, **caractérisée en ce qu'**elle contient au moins une autre substance filtre des UV, choisie dans le groupe formé par les triazines, les benzotriazoles et les pigments organiques et/ou inorganiques.

5. Préparation selon l'une quelconque des revendications précédentes, **caractérisée en ce qu'**elle contient au moins une autre substance filtre des UV-A et/ou un filtre à large bande, choisi(e)(s) dans le groupe formé par les dérivés de dibenzoylméthane [en particulier le 4-(tert-butyl)-4'-méthoxydibenzoylméthane] et la 2,4-bis-{[4·(2-éthylhexyloxy)-2-hydroxy]-phényl}-6-(4-méthoxyphényl)-1,3,5-triazine, les autres substances filtre pouvant à chaque fois être présentes seules ou dans des combinaisons quelconques les unes avec les autres.
